# EUROPEAN PATENT APPLICATION

(11) **EP 4 613 203 A1**
(43) Date of publication of application: **10.09.2025**
(21) Application number: 25160379.1
(22) Date of filing: 26.02.2025
(51) Int. Cl.: A61B 6/08, A61B 5/00, A61B 5/107

(54) **INFORMATION PROCESSING APPARATUS, RADIOGRAPHY SYSTEM, INFORMATION PROCESSING METHOD, AND PROGRAM**

(30) Priority: 05.03.2024 JP 2024033238
(71) Applicant: FUJIFILM Corporation, Tokyo 106-8620 (JP)
(72) Inventor: HOKIGUCHI, Tatsunari, Tokyo, 106-8620 (JP); ANDO, Yoshinori, Tokyo, 106-8620 (JP)
(74) Representative: Dehns Germany Partnerschaft mbB

(57) **Abstract**

Provided are an information processing apparatus, a radiography system, an information processing method, and a program that can support posture determination of a subject during radiography targeting a lung field.

The information processing apparatus includes at least one processor. The processor is configured to specify positions of left and right elbows of a subject and a position of a reference point on a back surface of the subject by using an optical image obtained by imaging the subject from a back; derive distances to the specified positions of the left and right elbows and a distance to the position of the reference point by using a distance image obtained by imaging the subject from the back; and perform a first determination on a posture of the subject based on the derived distances to the positions of the left and right elbows and the derived distance to the position of the reference point.

## Description

### BACKGROUND OF THE INVENTION

### 1. Field of the Invention

The disclosed technology relates to an information processing apparatus, a radiography system, an information processing method, and a program.

### 2. Description of the Related Art

The following technology is known for radiography. For example, JP2018-038908A describes a medical image diagnostic apparatus comprising an image acquisition unit that acquires an optical image of a subject, a position calculation unit that acquires three-dimensional position information of a characteristic part of the subject based on the optical image, and a controller that calculates an amount of movement of a top plate on which the subject is placed such that the characteristic part of the subject is moved to an imaging position of an imaging unit based on the position information of the characteristic part and moves the top plate based on the calculated amount of movement.

JP2021-056414A discloses a positioning system comprising an operable support that moves an arm and a leg of a patient, a sensor that provides a sensor signal indicating a body parameter of the patient, and a controller that determines a value of the body parameter based on the sensor signal and operates the support according to the determined value so that the patient is guided to a posture for diagnostic imaging.

### SUMMARY OF THE INVENTION

In a case in which radiography is performed on a lung field, in order to exclude a scapula from a lung field region, a subject is positioned to take a posture in which both elbows protrude forward with respect to a back surface. In a case where the protrusion of both elbows with respect to the back surface is insufficient, there is a risk that an image of the scapula is reflected in the lung field region. In this case, re-imaging is required, which leads to an increase in a burden on the radiologist and an increase in an amount of radiation exposure to the subject.

Currently, the determination of whether or not the posture of the subject is appropriate at the time of imaging is performed by the radiologist based on his/her own experience, and there are a certain number of cases in which imaging is performed in a state in which the posture of the subject is inappropriate, and the image of the scapula is reflected in the lung field region.

The disclosed technology has been made in view of the above points, and an object of the disclosed technology is to support posture determination of the subject during radiography targeting a lung field.

According to the disclosed technology, there is provided an information processing apparatus comprising at least one processor. The processor is configured to specify positions of left and right elbows of a subject and a position of a reference point on a back surface of the subject by using an optical image obtained by imaging the subject from a back; derive distances to the specified positions of the left and right elbows and a distance to the position of the reference point by using a distance image obtained by imaging the subject from the back; and perform a first determination on a posture of the subject based on the derived distances to the positions of the left and right elbows and the derived distance to the position of the reference point.

The processor may derive a difference between the distances to the positions of the left and right elbows and the distance to the position of the reference point; and perform the first determination by comparing the difference with a threshold value. The processor may output determination information indicating that the posture of the subject is appropriate in a case where the difference is equal to or greater than the threshold value. The processor may output determination information indicating that the posture of the subject is inappropriate in a case where the difference is less than the threshold value.

The processor may set the threshold value based on physique information indicating a physique of the subject. The processor may generate the physique information based on at least one of the optical image or the distance image.

The processor may specify positions of left and right shoulders of the subject by using the optical image; and specify a position of a center point of a line segment connecting the specified positions of the left and right shoulders as the position of the reference point.

The processor is may specify positions of left and right shoulders of the subject by using the optical image obtained by imaging the subject from the back; derive distances to the specified positions of the left and right shoulders by using the distance image obtained by imaging the subject from the back; and perform a second determination on a posture of the subject based on the derived distances to the positions of the left and right shoulders.

A radiography system according to the disclosed technology includes the information processing apparatus described above, an optical camera that generates the optical image; a distance sensor that generates the distance image; and a radiation source unit that emits radiation.

The optical camera and the distance sensor may be attached to the radiation source unit. The optical camera and the distance sensor may be integrally configured. The distance sensor may be a ToF camera or a stereo camera.

An information processing method according to the disclosed technology is a method executed by at least one processor included in an information processing apparatus, the method comprises specifying positions of left and right elbows of a subject and a position of a reference point on a back surface of the subject by using an optical image obtained by imaging the subject from a back; deriving distances to the specified positions of the left and right elbows and a distance to the position of the reference point by using a distance image obtained by imaging the subject from the back; and performing a first determination on a posture of the subject based on the derived distances to the positions of the left and right elbows and the derived distance to the position of the reference point.

A program according to the disclosed technology is a program for causing at least one processor included in an information processing apparatus to execute processing of specifying positions of left and right elbows of a subject and a position of a reference point on a back surface of the subject by using an optical image obtained by imaging the subject from a back; deriving distances to the specified positions of the left and right elbows and a distance to the position of the reference point by using a distance image obtained by imaging the subject from the back; and performing a first determination on a posture of the subject based on the derived distances to the positions of the left and right elbows and the derived distance to the position of the reference point.

According to the disclosed technology, it is possible to support posture determination of the subject during radiography targeting a lung field.

### BRIEF DESCRIPTION OF THE DRAWINGS

Fig. 1 is a diagram showing an example of a configuration of a radiography system according to an embodiment of the disclosed technology.
Fig. 2 is a diagram showing an example of a configuration of a radiography apparatus according to the embodiment of the disclosed technology.
Fig. 3 is a diagram showing an example of a hardware configuration of an information processing apparatus according to the embodiment of the disclosed technology.
Fig. 4 is a functional block diagram showing an example of a functional configuration of the information processing apparatus according to the embodiment of the disclosed technology.
Fig. 5 is a diagram showing an example of an aspect of processing of specifying each position of a body of a subject by a position specifying unit according to the embodiment of the disclosed technology.
Fig. 6 is a flowchart showing an example of a flow of processing performed by executing a processing program by a CPU of the information processing apparatus according to the embodiment of the disclosed technology.
Fig. 7 is a functional block diagram showing an example of a functional configuration of an information processing apparatus according to another embodiment of the disclosed technology.
Fig. 8 is a flowchart showing an example of a flow of processing performed by executing a processing program by a CPU of the information processing apparatus according to another embodiment of the disclosed technology.

### DESCRIPTION OF THE PREFERRED EMBODIMENTS

An example of embodiments of the disclosed technology will be described below with reference to the drawings. In addition, the same or equivalent components and parts in each drawing are given the same reference numerals, and duplicated descriptions will be omitted.

### First Embodiment

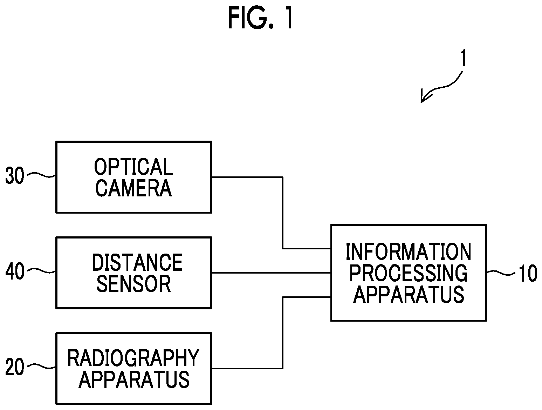

Fig. 1 is a diagram showing an example of a configuration of a radiography system 1 according to an embodiment of the disclosed technology. The radiography system 1 includes an information processing apparatus 10, a radiography apparatus 20, an optical camera 30, and a distance sensor 40.

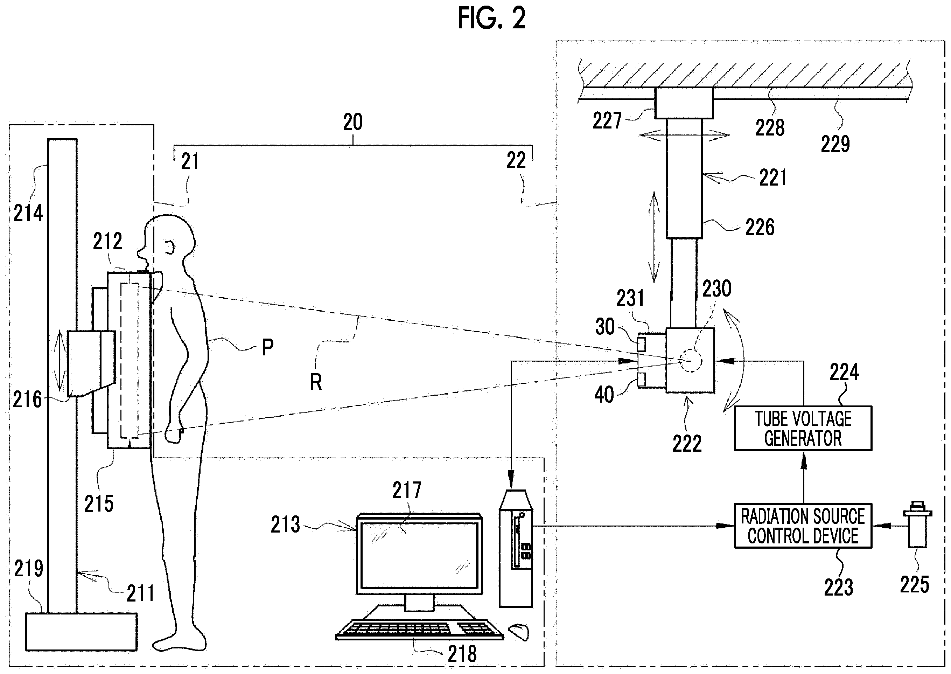

Fig. 2 is a diagram showing an example of the configuration of the radiography apparatus 20. The radiography apparatus 20 can perform radiography on the subject P by using radiation R such as X-rays. The radiography apparatus 20 is configured to include an imaging unit 21 and a radiation generation unit 22. The imaging unit 21 includes an upright imaging stand 211, a radiation detector 212, and a console 213. The radiation generation unit 22 has a radiation source suspension device 221, a radiation source unit 222, a radiation source control device 223, a tube voltage generator 224, and an irradiation switch 225.

The upright imaging stand 211 is an imaging stand for imaging the subject P in an upright posture. The upright imaging stand 211 has a pedestal 219 installed on a floor surface, a support column 214 extending from the pedestal 219 in a height direction, and a holder 215 that holds the radiation detector 212 inside. The holder 215 is connected to the support column 214 via a connecting portion 216. The connecting portion 216 and the holder 215 can be moved up and down along the support column 214. The holder 215 can be moved up and down through the console 213.

The radiation detector 212 generates a radiation image corresponding to the radiation R transmitted through the subject P. The radiation detector 212 is connected to the console 213 in a communicable manner by wire or wirelessly. The radiation detector 212 is used in a state of being accommodated in the holder 215.

The radiation detector 212 has a detection panel in which a plurality of pixels that accumulate charge corresponding to the radiation R are arranged in a two-dimensional matrix. The detection panel is also called a flat panel detector (FPD). In a case in which the irradiation with the radiation R is started, the detection panel starts an accumulation operation of accumulating the charge in the pixel. In a case in which the irradiation with the radiation R ends, the detection panel starts a readout operation of reading out the charge accumulated in the pixel as an electric signal.

The console 213 is, for example, a desktop, notebook, or tablet computer. The console 213 includes a display 217 that displays various screens and an input device 218 including a keyboard, a mouse, and the like. The console 213 transmits various signals to the radiation detector 212. In addition, the console 213 receives the radiation image from the radiation detector 212. The console 213 displays the radiation image on the display 217.

The radiation source suspension device 221 has an arm 226 and a carriage 227. The radiation source unit 222 is attached to a distal end of the arm 226, and a proximal end of the arm 226 is attached to the carriage 227. The arm 226 is expandable and contractible in the vertical direction by a motor or the like. A height position of the radiation source unit 222 can be changed by expanding and contracting the arm 226 in the vertical direction. The radiation source unit 222 is rotatable about an axis perpendicular to the expansion and contraction direction of the arm 226. The expansion and contraction of the arm 226 and the rotation of the radiation source unit 222 can be performed through the console 213.

The carriage 227 is connected to a rail 229 laid on the ceiling 228. That is, the radiation source unit 222 is a ceiling suspension type. The radiation source unit 222 can be moved parallel along the rail 229. As the radiation source unit 222 is moved parallel along the rail 229, a source to image receptor distance (SID) which is a distance from the radiation source unit 222 to a detection surface of the radiation detector 212 is changed. The parallel movement of the radiation source unit 222 can be performed through the console 213.

The radiation source unit 222 includes a radiation tube 230 and a collimator 231. The radiation tube 230 is provided with a filament, a target, a grid electrode, and the like (all are not illustrated). A voltage is applied between the filament which is a cathode, and the target which is an anode. The voltage to be applied between the filament and the target is referred to as a tube voltage. The filament releases thermal electrons according to the applied tube voltage toward the target. The target emits the radiation R with collision of the thermal electrons from the filament. The grid electrode is disposed between the filament and the target. The grid electrode changes a flow rate of the thermal electrons from the filament toward the target according to the applied voltage. The flow rate of the thermal electrons from the filament toward the target is referred to as a tube current.

The collimator 231 limits an irradiation range of the radiation R emitted from the radiation tube 230. The collimator 231 is composed of a radiation-shielding member such as lead and has four shielding plates disposed on each side of a quadrangle. The radiation R emitted from the radiation tube 230 is transmitted through an opening portion formed at a center of the four shielding plates and irradiates the subject P. The size of the opening portion is changed by changing the position of each of the shielding plates, and thus the irradiation range of the radiation R is changed.

The tube voltage generator 224 and the irradiation switch 225 are connected to the radiation source control device 223. The radiation source control device 223 controls the operation of the radiation source unit 222 in response to various command signals from the irradiation switch 225. The irradiation switch 225 is operated in a case in which the irradiation of the radiation R is started.

In a case in which the radiography is performed on the lung field of the subject P, as shown in Fig. 2, the subject P is positioned such that the chest of the subject P is in contact with a front surface of the holder 215. The radiation R is emitted from the back of the subject P.

The optical camera 30 is an imaging device capable of generating an optical image (RGB image). An optical image of the subject P is captured by the optical camera 30. The optical camera 30 is attached to the radiation source unit 222. The entire irradiation range of the radiation R at the position of the subject P is included in the imaging range of the optical camera 30. In a case in which the radiography is performed on the lung field of the subject P, the optical image is captured from the back of the subject P.

The distance sensor 40 is a device that can generate a distance image representing a distance to a surface of an object. A distance image of the subject P is captured by the distance sensor 40. The distance sensor 40 may be, for example, a time-of-flight (ToF) camera that generates a distance image by ToF method using infrared light. In addition, the distance sensor 40 may be a stereo camera that generates a distance image based on a principle of triangulation using two cameras. The distance sensor 40 is attached to the radiation source unit 222, similarly to the optical camera 30. In a case in which the radiography is performed on the lung field of the subject P, the distance image is captured from the back of the subject P. In the present embodiment, although a case where the optical camera 30 and the distance sensor 40 are configured as separate devices is illustrated, the optical camera 30 and the distance sensor 40 may be configured integrally. That is, it is possible to use a single device capable of performing both the capturing of the optical image and the capturing of the distance image.

The information processing apparatus 10 has a function of determining a posture of the subject P in a case where radiography is performed on the lung field of the subject P based on the optical image of the subject P output from the optical camera 30 and a distance image of the subject P output from the distance sensor 40. The information processing apparatus 10 may also serve as the console 213.

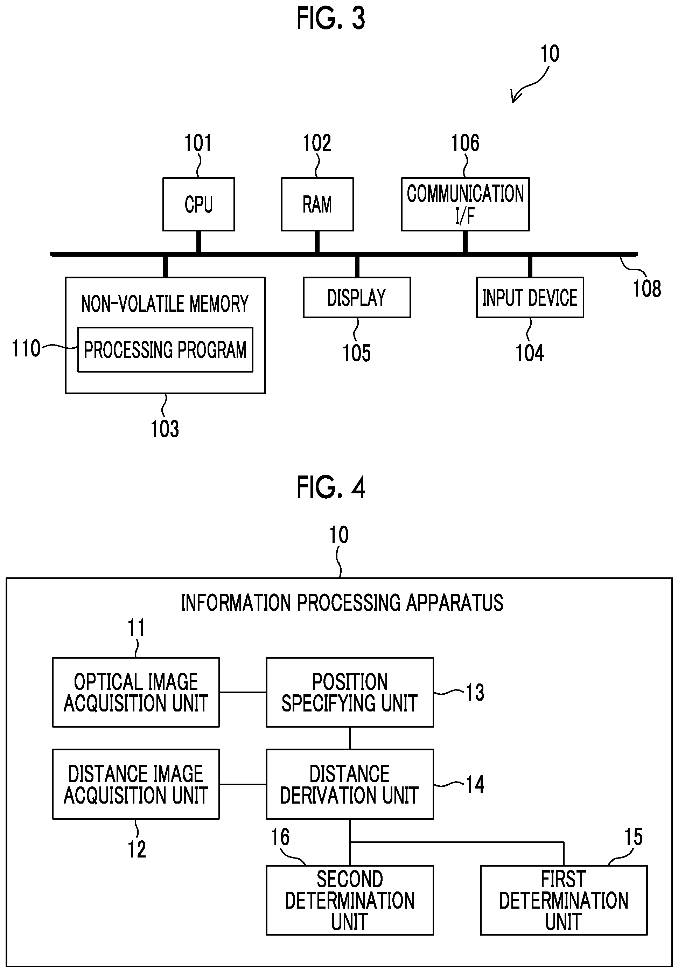

Fig. 3 is a diagram showing an example of a hardware configuration of the information processing apparatus 10. The information processing apparatus 10 includes a central processing unit (CPU) 101, a random-access memory (RAM) 102, a non-volatile memory 103, an input device 104 including a keyboard and a mouse, a display 105, and a communication interface 106. These pieces of hardware are connected to a bus 108.

The display 105 may be a touch panel display. The communication interface 106 is an interface for the information processing apparatus 10 to perform communication with the optical camera 30, the distance sensor 40, and the radiography apparatus 20. The communication method may be either wired or wireless. For the wireless communication, it is possible to apply a method conforming to existing wireless communication standards such as, for example, Wi-Fi (registered trademark) and Bluetooth (registered trademark).

The non-volatile memory 103 is a non-volatile storage medium such as a hard disk or a flash memory. A processing program 110 is stored in the non-volatile memory 103. The RAM 102 is a work memory for the CPU 101 to execute processing. The CPU 101 loads the processing program 110 stored in the non-volatile memory 103 into the RAM 102 and executes processing according to the processing program 110. The CPU 101 is an example of a "processor" in the disclosed technology.

Fig. 4 is a functional block diagram showing an example of a functional configuration of the information processing apparatus 10. The information processing apparatus 10 includes an optical image acquisition unit 11, a distance image acquisition unit 12, a position specifying unit 13, a distance derivation unit 14, a first determination unit 15, and a second determination unit 16. By executing the processing program 110 by the CPU 101, the information processing apparatus 10 functions as the optical image acquisition unit 11, the distance image acquisition unit 12, the position specifying unit 13, the distance derivation unit 14, the first determination unit 15, and the second determination unit 16.

The optical image acquisition unit 11 acquires an optical image of the subject P captured from the back, which is output from the optical camera 30. The distance image acquisition unit 12 acquires the distance image of the subject P captured from the back, which is output from the distance sensor 40. In a case in which the radiography is performed on the lung field of the subject P, in order to exclude the scapula from the lung field region, the subject P is positioned such that the chest comes into contact with the front surface of the holder 215 that holds the radiation detector 212 inside and takes a posture in which both elbows protrude forward with respect to the back surface. The optical image acquisition unit 11 and the distance image acquisition unit 12 acquire the optical image and the distance image of the subject P in a state in which the positioning described above is performed.

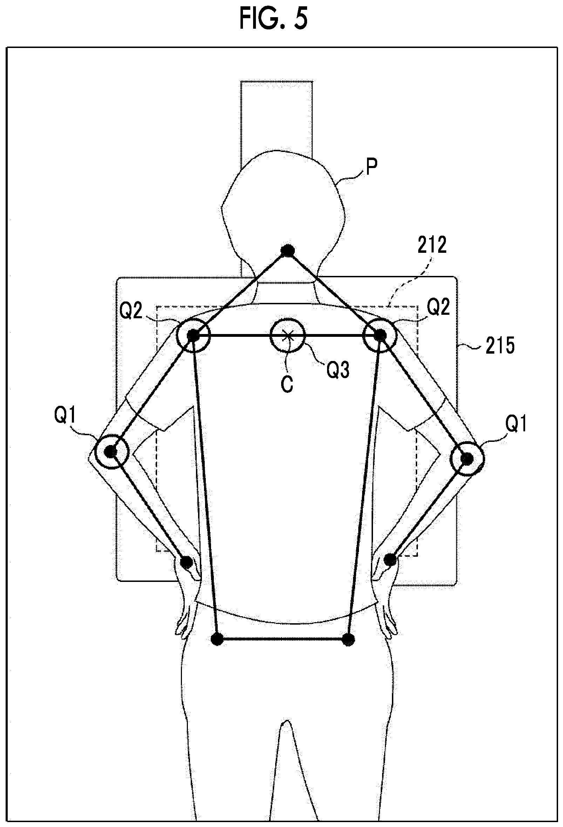

The position specifying unit 13 specifies positions of the left and right elbows of the subject P, positions of the left and right shoulders of the subject P, and a position of the reference point on the back surface of the subject P from the optical image acquired by the optical image acquisition unit 11. Fig. 5 is a diagram showing an example of an aspect of processing of the position specifying unit 13 specifying positions Q1 of the left and right elbows of the subject P, positions Q2 of the left and right shoulders of the subject P, and a position Q3 of the reference point on the back surface of the subject P.

The position specifying unit 13 specifies positions Q1 of the left and right elbows of the subject P, positions Q2 of the left and right shoulders of the subject P, and a position Q3 of the reference point using a known posture estimation technique. The posture estimation technique is a technique of estimating a human posture by extracting feature points of eyes, ears, a nose, an elbow, a shoulder, a wrist, a knee, and the like from an image obtained by imaging a human body using a model trained by machine learning.

The position specifying unit 13 specifies positions Q1 of the left and right elbows of the subject P from the extraction results of both elbows, and specifies positions Q2 of the left and right shoulders of the subject P from the extraction results of both shoulders. The position specifying unit 13 specifies a position of a center point C of a line segment connecting the specified positions Q2 of the left and right shoulders as a position Q3 of a reference point on the back surface of the subject P. In the present embodiment, the center point C of the line segment connecting the positions of the left and right shoulders is used as the reference point, but any point located at the center of the back surface of the subject P in the left-right direction can be used as the reference point.

The distance derivation unit 14 derives distances L1 to the positions Q1 of the left and right elbows of the subject P, distances L2 to the positions Q2 of the left and right shoulders of the subject P, and a distance L3 to a position Q3 of the reference point on the back surface of the subject P by using the distance image acquired by the distance image acquisition unit 12. The distance derivation unit 14 converts the coordinate positions of the positions Q1, Q2, and Q3 specified by the position specifying unit 13 on the optical image into coordinate positions on the distance image. This coordinate transformation can be performed by geometric calculation based on the installation positions of the optical camera 30 and the distance sensor 40. The distance derivation unit 14 derives distances L1 to the positions Q1 of the left and right elbows of the subject P, distances L2 to the positions Q2 of the left and right shoulders of the subject P, and a distance L3 to the position Q3 of the reference point on the back surface of the subject P, from a pixel value or a three-dimensional coordinate value corresponding to the pixel value at the coordinate positions of the positions Q1, Q2, and Q3 on the distance image.

The first determination unit 15 performs the main determination on the posture of the subject P based on the distances L1 to the positions Q1 of the left and right elbows of the subject P and the distance L3 to the position Q3 of the reference point derived by the distance derivation unit 14. Specifically, the first determination unit 15 derives a difference ΔL_{A} (= L1 - L3) between the distances L1 to the positions Q1 of the left and right elbows and the distance L3 to the position of the reference point Q3, and performs the main determination on the posture of the subject P by comparing the difference ΔL_{A} with the threshold value T1. The main determination is an example of a "first determination" in the disclosed technology. The derivation of the difference ΔL_{A} is performed for each of the left and right elbows, and the comparison with the threshold value T1 is performed for each of the differences ΔL_{A} derived for each of the left and right elbows. In the present embodiment, the threshold value T1 is a predetermined value. The threshold value T1 is set in consideration of an amount required as an amount of protrusion of both elbows forward with respect to the back surface. For example, the threshold value T1 may be determined based on data in a case where the radiography is performed on the lung fields of a plurality of subjects. For example, the threshold value T1 can be set to 20 mm.

In a case where the difference ΔL_{A} is equal to or greater than the threshold value T1 (ΔL_{A} ≥ T1), the first determination unit 15 outputs determination information indicating that the posture of the subject P is appropriate. In a case in which radiography is performed on a lung field, it is necessary to perform the imaging in a posture in which both elbows protrude forward with respect to the back surface in order to exclude the scapula from the lung field region. The fact that the difference ΔL_{A} is equal to or greater than the threshold value T1 means that the posture of the subject P is an appropriate posture in which both elbows protrude forward with respect to the back surface. Therefore, in a case where ΔL_{A} ≥ T1 is established for each of the left and right elbows, the first determination unit 15 outputs determination information indicating that the posture of the subject P is appropriate.

On the other hand, in a case where the difference ΔL_{A} is less than the threshold value T1 (ΔL_{A} < T1), the first determination unit 15 outputs determination information indicating that the posture of the subject P is inappropriate. The fact that the difference ΔL_{A} is less than the threshold value T1 means that the posture of the subject P is an inappropriate posture in which both elbows do not protrude forward with respect to the back surface or the amount of protrusion of both elbows is insufficient. Therefore, in a case in which ΔL_{A} < T1 is established for at least one of the left or right elbow, the first determination unit 15 outputs determination information indicating that the posture of the subject P is inappropriate. In this case, the first determination unit 15 may include information indicating which of the left and right elbows is in the state of ΔL_{A} < T1 in the determination information.

The output of the determination information related to the main determination may be performed by, for example, display on the display 105, output of a voice, light emission of a lamp, or the like. In addition, in a case in which the radiography apparatus 20 comprises a display panel, the determination information related to the main determination may be output by displaying the determination information on the display panel. The first determination unit 15 may output only one of the determination information indicating that the posture of the subject P is appropriate or the determination information indicating that the posture of the subject P is inappropriate.

The second determination unit 16 performs a preliminary determination on the posture of the subject P based on the distances L2 to the positions Q2 of the left and right shoulders of the subject P derived by the distance derivation unit 14. Specifically, the second determination unit 16 derives a difference ΔL_{B} (= |L2_{R} - L2_{L}|) between a distance L2_{R} to a position of the right shoulder of the subject P and a distance L2_{L} to a position of the left shoulder of the subject P, and performs a preliminary determination on the posture of the subject P by comparing the difference ΔL_{B} with a threshold value T2. The preliminary determination by the second determination unit 16 is executed before the main determination by the first determination unit 15. The preliminary determination is a determination as to whether or not the subject P is facing the detection surface of the radiation detector 212. The preliminary determination is an example of a "second determination" in the disclosed technology.

In a case where the difference ΔL_{B} is less than the threshold value T2 (ΔL_{B} < T2), the second determination unit 16 outputs determination information indicating that the posture of the subject P is appropriate. The fact that the difference ΔL_{B} is less than the threshold value T2 means that the posture of the subject P is an appropriate posture in which the subject P faces the detection surface of the radiation detector 212. Therefore, in this case, the second determination unit 16 outputs the determination information indicating that the posture of the subject P is appropriate.

On the other hand, in a case where the difference ΔL_{B} is equal to or greater than the threshold value T2 (ΔL_{B} ≥ T2), the second determination unit 16 outputs determination information indicating that the posture of the subject P is appropriate. The fact that the difference ΔL_{B} is equal to or greater than the threshold value T2 means that the posture of the subject P is an inappropriate posture in which the subject P does not face the detection surface of the radiation detector 212. Therefore, in this case, the second determination unit 16 outputs the determination information indicating that the posture of the subject P is inappropriate.

The output of the determination information related to the preliminary determination may be performed by, for example, display on the display 105, output of a voice, light emission of a lamp, or the like. In addition, in a case in which the radiography apparatus 20 comprises a display panel, the determination information related to the preliminary determination may be output by displaying the determination information on the display panel. In addition, the second determination unit 16 may output only one of the determination information indicating that the posture of the subject P is appropriate or the determination information indicating that the posture of the subject P is inappropriate.

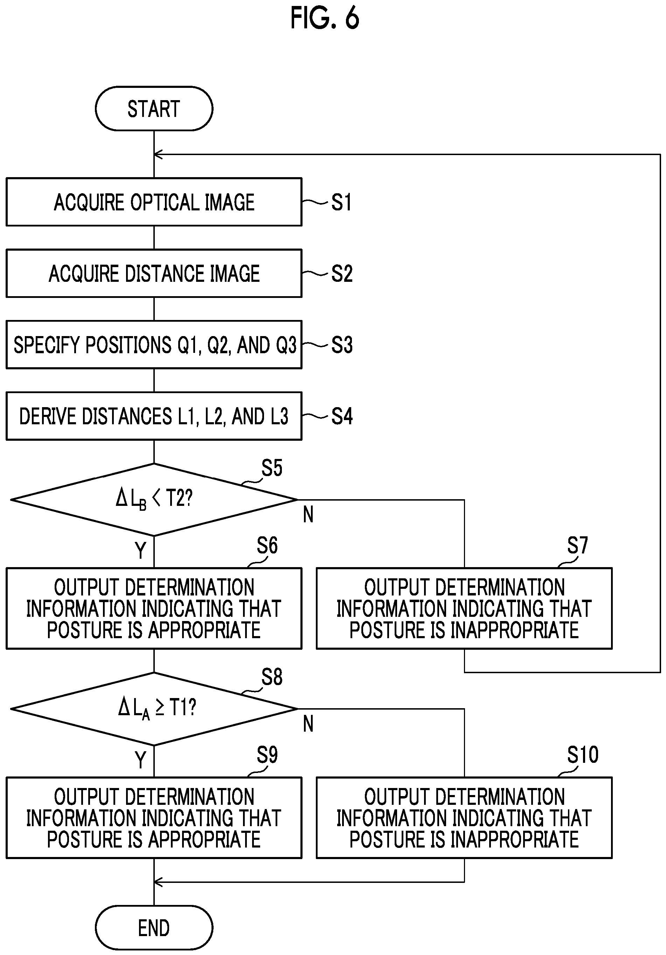

Fig. 6 is a flowchart showing an example of a flow of processing performed by the CPU 101 executing the processing program 110.

In step S1, the optical image acquisition unit 11 acquires the optical image of the subject P captured from the back, which is output from the optical camera 30.

In step S2, the distance image acquisition unit 12 acquires the distance image of the subject P captured from the back, which is output from the distance sensor 40.

In step S3, the position specifying unit 13 specifies positions Q1 of the left and right elbows of the subject P and positions Q2 of the left and right shoulders of the subject P from the optical image acquired in step S1. Subsequently, the position specifying unit 13 specifies a position of a center point C of a line segment connecting the specified positions Q2 of the left and right shoulders as a position Q3 of a reference point on the back surface of the subject P.

In step S4, the distance derivation unit 14 derives distances L1 to the positions Q1 of the left and right elbows of the subject P specified in step S3 by using the distance image acquired in step S2. In addition, the distance derivation unit 14 derives distances L2 to the positions Q2 of the left and right shoulders of the subject P specified in step S3 by using the distance image acquired in step S2. Further, the distance derivation unit 14 derives the distance L3 to the position Q3 of the reference point specified in step S3 by using the distance image acquired in step S2.

In step S5, the second determination unit 16 performs the preliminary determination on the posture of the subject P based on the distance L2 derived in step S4. Specifically, the second determination unit 16 derives a difference ΔL_{B} (= |L2_{R} - L2_{L}|) between a distance L2_{R} to the position of the right shoulder of the subject P and a distance L2_{L} to the position of the left shoulder of the subject P, and determines whether or not the difference ΔL_{B} is less than the threshold value T2. In a case where it is determined that the difference ΔL_{B} is less than the threshold value T2, the processing proceeds to step S6, and in a case where it is determined that the difference ΔL_{B} is equal to or greater than the threshold value T2, the processing proceeds to step S7.

In step S6, the second determination unit 16 outputs determination information indicating that the posture of the subject P is appropriate. The fact that the difference ΔL_{B} is less than the threshold value T2 means that the posture of the subject P is an appropriate posture in which the subject P faces the detection surface of the radiation detector 212. Therefore, in this case, the second determination unit 16 outputs the determination information indicating that the posture of the subject P is appropriate. Then, the processing proceeds to step S8.

In step S7, the second determination unit 16 outputs determination information indicating that the posture of the subject P is inappropriate. The fact that the difference ΔL_{B} is equal to or greater than the threshold value T2 means that the posture of the subject P is an inappropriate posture in which the subject P does not face the detection surface of the radiation detector 212. Therefore, in this case, the second determination unit 16 outputs the determination information indicating that the posture of the subject P is inappropriate. The posture of the subject P is corrected based on the determination information. Then, the processing returns to step S1, and the determination processing is performed again by the second determination unit 16 for the corrected posture of the subject P.

In step S8, the first determination unit 15 performs the main determination on the posture of the subject P based on the distance L1 and the distance L3 derived in step S4. Specifically, the first determination unit 15 derives a difference ΔL_{A} (= L1 - L3) between the distances L1 to the positions Q1 of the left and right elbows and the distance L3 to the position of the reference point Q3, and determines whether or not the difference ΔL_{A} is equal to or greater than the threshold value T1. In a case where it is determined that the difference ΔL_{A} is equal to or greater than the threshold value T1, the processing proceeds to step S9, and in a case where it is determined that the difference ΔL_{A} is less than the threshold value T1, the processing proceeds to step S10.

In step S9, the first determination unit 15 outputs determination information indicating that the posture of the subject P is appropriate. The fact that the difference ΔL_{A} is equal to or greater than the threshold value T1 means that the posture of the subject P is an appropriate posture in which both elbows protrude forward with respect to the back surface. Therefore, in this case, the first determination unit 15 outputs the determination information indicating that the posture of the subject P is appropriate.

In step S10, the first determination unit 15 outputs determination information indicating that the posture of the subject P is inappropriate. The fact that the difference ΔL_{A} is less than the threshold value T1 means that the posture of the subject P is an inappropriate posture in which both elbows do not protrude forward with respect to the back surface or the amount of protrusion of both elbows is insufficient. Therefore, in this case, the first determination unit 15 outputs the determination information indicating that the posture of the subject P is inappropriate.

In a case in which radiography (chest X-ray) is performed on a lung field, in order to exclude the scapula from the lung field region, the subject needs to take a posture in which both elbows protrude forward with respect to a back surface. In a case in which the protrusion of both elbows with respect to the back surface is insufficient, the image of the scapula may be reflected in the lung field region of the radiation image. In this case, re-imaging is required, which leads to an increase in a burden on the radiologist and an increase in an amount of radiation exposure to the subject.

Currently, the determination of whether or not the posture of the subject is appropriate at the time of imaging is performed by the radiologist based on his/her own experience, and there are a certain number of cases in which imaging is performed in a state in which the posture of the subject is inappropriate, and the image of the scapula is reflected in the lung field region.

With the information processing apparatus 10 according to the embodiment of the disclosed technology, determination is made as to whether or not the subject is in an appropriate posture in which the left and right elbows protrude forward with respect to the back surface. Therefore, it is possible to support the determination of the posture of the subject during radiography targeting a lung field.

### Second Embodiment

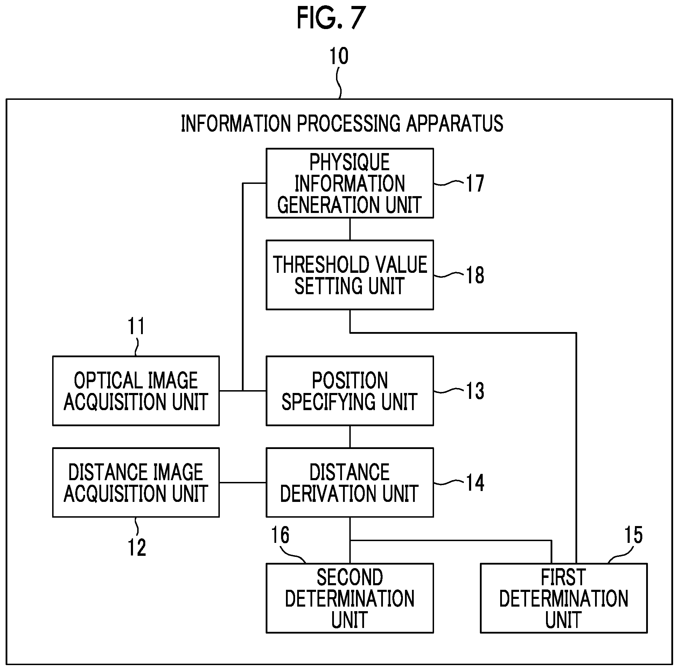

Fig. 7 is a functional block diagram showing an example of a functional configuration of the information processing apparatus 10 according to a second embodiment of the disclosed technology. The information processing apparatus 10 according to the second embodiment is different from the information processing apparatus 10 according to the first embodiment in that a physique information generation unit 17 and a threshold value setting unit 18 are further provided.

The physique information generation unit 17 generates physique information indicating the physique of the subject P based on the optical image of the subject P captured from the back, which is acquired by the optical image acquisition unit 11. The physique information may be a numerical value indicating at least one of a height, a weight, or a chest circumference of the subject P estimated from the optical image obtained by imaging the subject P. In addition, the physique information may be information indicating a classification of the physique of the subject P (standard, large, small, thin, or obese).

The threshold value setting unit 18 sets the threshold value T1 used in the main determination by the first determination unit 15 based on the physique information generated by the physique information generation unit 17. For example, the threshold value setting unit 18 may set a larger value as the threshold value T1 as the physique indicated by the physique information is larger.

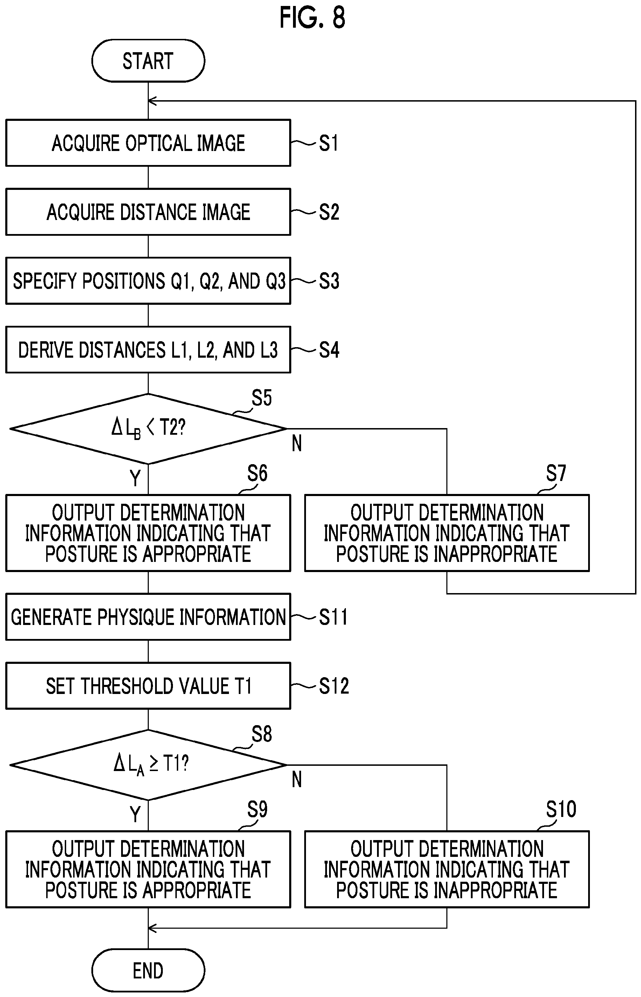

Fig. 8 is a flowchart showing an example of a flow of processing performed by executing a processing program 110 by a CPU 101 of the information processing apparatus 10 according to the second embodiment. The processing flow according to the second embodiment is obtained by adding processing of steps S11 and S12 to the processing flow according to the first embodiment.

The processing of step S11 is executed after the processing of step S6 (output of the determination information related to the preliminary determination). In step S11, the physique information generation unit 17 generates physique information indicating the physique of the subject P based on the optical image acquired in step S1.

In step S12, the threshold value setting unit 18 sets the threshold value T1 used in the main determination by the first determination unit 15 based on the physique information generated in step S11. After the processing of step S12, the processing of step S8 is executed.

In step S8, the first determination unit 15 derives a difference ΔL_{A} (= L1 - L3) between the distances L1 to the positions Q1 of the left and right elbows and the distance L3 to the position of the reference point Q3, and determines whether or not the difference ΔL_{A} is equal to or greater than the threshold value T1 set in step S12.

As described above, with the information processing apparatus 10 according to the second embodiment, the threshold value T1 used in the main determination (determination of whether or not the appropriate posture in which the left and right elbows protrude forward with respect to the back surface is achieved) by the first determination unit 15 is set according to the physique of the subject P. Therefore, it is possible to perform more appropriate determination.

In the above description, a case where the physique information is generated based on the optical image has been exemplified, but the disclosed technology is not limited to this aspect. The physique information generation unit 17 may generate physique information indicating the physique of the subject P based on the distance image of the subject P captured from the back, which is acquired by the distance image acquisition unit 12. In addition, the physique information generation unit 17 may generate the physique information based on both the optical image and the distance image. In addition, in a case where the physique information of the subject P is known, the threshold value setting unit 18 may set the threshold value T1 based on the known physique information. In this case, the physique information generation unit 17 is not necessary.

In each of the above-described embodiments, for example, as a hardware structure of processing units that execute various types of processing, such as the optical image acquisition unit 11, the distance image acquisition unit 12, the position specifying unit 13, the distance derivation unit 14, the first determination unit 15, the second determination unit 16, the physique information generation unit 17, and the threshold value setting unit 18, various processors shown below can be used. The above-described various processors include, for example, a programmable logic device (PLD) which is a processor having a changeable circuit configuration after manufacturing, such as an FPGA, and a dedicated electrical circuit which is a processor having a dedicated circuit configuration designed to execute specific processing, such as an application specific integrated circuit (ASIC), in addition to the GPU and the CPU which is a general-purpose processor that executes software (programs) to function as various processing units, as described above.

One processing unit may be configured by one of the various processors, or may be configured by a combination of the same or different types of two or more processors (for example, a combination of a plurality of FPGAs or a combination of the CPU and the FPGA). In addition, a plurality of processing units may be configured by one processor.

As an example in which a plurality of processing units are configured by one processor, first, there is a form in which one processor is configured by a combination of one or more CPUs and software as typified by a computer, such as a client or a server, and this processor functions as a plurality of processing units. Second, as represented by a system on a chip (SoC) or the like, there is a form in which a processor that realizes functions of an entire system including a plurality of processing units with one integrated circuit (IC) chip is used. In this way, various processing units are configured by one or more of the above-described various processors as hardware structures.

Furthermore, as the hardware structure of the various processors, more specifically, an electrical circuit (circuitry) in which circuit elements such as semiconductor elements are combined can be used.

Moreover, in the embodiments described above, the aspect has been described in which the processing program 110 is stored (installed) in advance in the non-volatile memory 103, but the disclosed technology is not limited to this. The processing program 110 may be provided in a form recorded in a recording medium such as a compact disc read-only memory (CD-ROM), a digital versatile disc read-only memory (DVD-ROM), and a universal serial bus (USB) memory. Alternatively, a form may be employed in which the above-described processing program 110 is downloaded from an external device via the network.

In regard to the first and second embodiments described above, the following supplementary note will be further disclosed.

### Supplementary Note 1

An information processing apparatus comprising at least one processor,
in which the processor is configured to:
specify positions of left and right elbows of a subject and a position of a reference point on a back surface of the subject by using an optical image obtained by imaging the subject from a back;
derive distances to the specified positions of the left and right elbows and a distance to the position of the reference point by using a distance image obtained by imaging the subject from the back; and
perform a first determination on a posture of the subject based on the derived distances to the positions of the left and right elbows and the derived distance to the position of the reference point.

### Supplementary Note 2

The information processing apparatus according to Supplementary Note 1,
in which the processor is configured to:
derive a difference between the distances to the positions of the left and right elbows and the distance to the position of the reference point; and
perform the first determination by comparing the difference with a threshold value.

### Supplementary Note 3

The information processing apparatus according to Supplementary Note 2,
in which the processor is configured to output determination information indicating that the posture of the subject is appropriate in a case where the difference is equal to or greater than the threshold value.

### Supplementary Note 4

The information processing apparatus according to Supplementary Note 2 or Supplementary Note 3,
in which the processor is configured to output determination information indicating that the posture of the subject is inappropriate in a case where the difference is less than the threshold value.

### Supplementary Note 5

The information processing apparatus according to any one of Supplementary Note 2 to Supplementary Note 4,
in which the processor is configured to set the threshold value based on physique information indicating a physique of the subject.

### Supplementary Note 6

The information processing apparatus according to Supplementary Note 5,
in which the processor is configured to generate the physique information based on at least one of the optical image or the distance image.

### Supplementary Note 7

The information processing apparatus according to any one of Supplementary Note 1 to Supplementary Note 6,
in which the processor is configured to:
specify positions of left and right shoulders of the subject by using the optical image; and
specify a position of a center point of a line segment connecting the specified positions of the left and right shoulders as the position of the reference point.

### Supplementary Note 8

The information processing apparatus according to any one of Supplementary Note 1 to Supplementary Note 7,
in which the processor is configured to:
specify positions of left and right shoulders of the subject by using the optical image obtained by imaging the subject from the back;
derive distances to the specified positions of the left and right shoulders by using the distance image obtained by imaging the subject from the back; and
perform a second determination on a posture of the subject based on the derived distances to the positions of the left and right shoulders.

### Supplementary Note 9

A radiography system comprising:
the information processing apparatus according to any one of Supplementary Note 1 to Supplementary Note 8;
an optical camera that generates the optical image;
a distance sensor that generates the distance image; and
a radiation source unit that emits radiation.

### Supplementary Note 10

The radiography system according to Supplementary Note 9,
in which the optical camera and the distance sensor are attached to the radiation source unit.

### Supplementary Note 11

The radiography system according to Supplementary Note 9 or Supplementary Note 10,
in which the optical camera and the distance sensor are integrally configured.

### Supplementary Note 12

The radiography system according to any one of Supplementary Note 9 to Supplementary Note 11;
in which the distance sensor is a ToF camera or a stereo camera.

### Supplementary Note 13

An information processing method executed by at least one processor included in an information processing apparatus, the method comprising:
specifying positions of left and right elbows of a subject and a position of a reference point on a back surface of the subject by using an optical image obtained by imaging the subject from a back;
deriving distances to the specified positions of the left and right elbows and a distance to the position of the reference point by using a distance image obtained by imaging the subject from the back; and
performing a first determination on a posture of the subject based on the derived distances to the positions of the left and right elbows and the derived distance to the position of the reference point.

### Supplementary Note 14

A program for causing at least one processor included in an information processing apparatus to execute processing of:
specifying positions of left and right elbows of a subject and a position of a reference point on a back surface of the subject by using an optical image obtained by imaging the subject from a back;
deriving distances to the specified positions of the left and right elbows and a distance to the position of the reference point by using a distance image obtained by imaging the subject from the back; and
performing a first determination on a posture of the subject based on the derived distances to the positions of the left and right elbows and the derived distance to the position of the reference point.

### Explanation of References

1: radiography system
10: information processing apparatus
11: optical image acquisition unit
12: distance image acquisition unit
13: position specifying unit
14: distance derivation unit
15: first determination unit
16: second determination unit
17: physique information generation unit
18: threshold value setting unit
20: radiography apparatus
21: imaging unit
22: radiation generation unit
30: optical camera
40: distance sensor
101: CPU
102: RAM
103: non-volatile memory
104: input device
105: display
106: communication interface
108: bus
110: processing program
211: upright imaging stand
212: radiation detector
213: console
214: support column
215: holder
216: connecting portion
217: display
218: input device
219: pedestal
221: radiation source suspension device
222: radiation source unit
223: radiation source control device
224: tube voltage generator
225: irradiation switch
226: arm
227: carriage
228: ceiling
229: rail
230: radiation tube
231: collimator

## Claims

1. An information processing apparatus comprising at least one processor,
wherein the processor is configured to:
specify positions of left and right elbows of a subject and a position of a reference point on a back surface of the subject by using an optical image obtained by imaging the subject from a back;
derive distances to the specified positions of the left and right elbows and a distance to the position of the reference point by using a distance image obtained by imaging the subject from the back; and
perform a first determination on a posture of the subject based on the derived distances to the positions of the left and right elbows and the derived distance to the position of the reference point.

2. The information processing apparatus according to claim 1,
wherein the processor is configured to:
derive a difference between the distances to the positions of the left and right elbows and the distance to the position of the reference point; and
perform the first determination by comparing the difference with a threshold value.

3. The information processing apparatus according to claim 2,
wherein the processor is configured to output determination information indicating that the posture of the subject is appropriate in a case where the difference is equal to or greater than the threshold value.

4. The information processing apparatus according to claim 2,
wherein the processor is configured to output determination information indicating that the posture of the subject is inappropriate in a case where the difference is less than the threshold value.

5. The information processing apparatus according to any one of claims 2 to 4,
wherein the processor is configured to set the threshold value based on physique information indicating a physique of the subject.

6. The information processing apparatus according to claim 5,
wherein the processor is configured to generate the physique information based on at least one of the optical image or the distance image.

7. The information processing apparatus according to any one of the preceding claims,
wherein the processor is configured to:
specify positions of left and right shoulders of the subject by using the optical image; and
specify a position of a center point of a line segment connecting the specified positions of the left and right shoulders as the position of the reference point.

8. The information processing apparatus according to any one of the preceding claims,
wherein the processor is configured to:
specify positions of left and right shoulders of the subject by using the optical image obtained by imaging the subject from the back;
derive distances to the specified positions of the left and right shoulders by using the distance image obtained by imaging the subject from the back; and
perform a second determination on a posture of the subject based on the derived distances to the positions of the left and right shoulders.

9. A radiography system comprising:
the information processing apparatus according to any one of claims 1 to 8;
an optical camera that generates the optical image;
a distance sensor that generates the distance image; and
a radiation source unit that emits radiation.

10. The radiography system according to claim 9,
wherein the optical camera and the distance sensor are attached to the radiation source unit.

11. The radiography system according to claim 9,
wherein the optical camera and the distance sensor are integrally configured.

12. The radiography system according to claim 9 or 10,
wherein the distance sensor is a ToF camera or a stereo camera.

13. An information processing method executed by at least one processor included in an information processing apparatus, the method comprising:
specifying positions of left and right elbows of a subject and a position of a reference point on a back surface of the subject by using an optical image obtained by imaging the subject from a back;
deriving distances to the specified positions of the left and right elbows and a distance to the position of the reference point by using a distance image obtained by imaging the subject from the back; and
performing a first determination on a posture of the subject based on the derived distances to the positions of the left and right elbows and the derived distance to the position of the reference point.

14. A program for causing at least one processor included in an information processing apparatus to execute processing of:
specifying positions of left and right elbows of a subject and a position of a reference point on a back surface of the subject by using an optical image obtained by imaging the subject from a back;
deriving distances to the specified positions of the left and right elbows and a distance to the position of the reference point by using a distance image obtained by imaging the subject from the back; and
performing a first determination on a posture of the subject based on the derived distances to the positions of the left and right elbows and the derived distance to the position of the reference point.
